# EUROPEAN PATENT SPECIFICATION

(11) **EP 4 427 743 B1**
(45) Date of publication and mention of the grant of the patent: **17.06.2026**
(21) Application number: 22890306.8
(22) Date of filing: 31.10.2022
(51) Int. Cl.: A61K 9/51, A61K 31/337, A61P 35/00, A61K 9/00

(54) **METHOD FOR PRODUCING ALBUMIN-BOUND TAXANE NANOPARTICLES WITH IMPROVED STABILITY IN MAINTAINING PARTICLE SIZE DISTRIBUTION**
VERFAHREN ZUR HERSTELLUNG ALBUMINGEBUNDENER TAXANNANOPARTIKEL MIT VERBESSERTER STABILITÄT BEI DER ERHALTUNG DER PARTIKELGRÖSSENVERTEILUNG
PROCÉDÉ DE PRODUCTION DE NANOPARTICULES DE TAXANE LIÉES À L'ALBUMINE PRÉSENTANT UNE STABILITÉ AMÉLIORÉE DANS LE MAINTIEN DE LA DISTRIBUTION DE LA TAILLE DES PARTICULES

(30) Priority: 03.11.2021 KR 20210150107
(43) Date of publication of application: 11.09.2024
(73) Proprietor: SNBioscience Inc., Seongnam-si, Gyeonggi-do, 13449 (KR)
(72) Inventor: PARK, Young Hwan, 06374 Seoul (KR); PARK, Sang Man, 1404 Gyeongsangnam-do (KR); HONG, Soon-Seok, 02232 Seoul (KR); CHO, Wooram, 13105 Gyeonggi-do (KR)
(74) Representative: J A Kemp LLP
(86) International application number: PCT/KR2022/016812
(87) International publication number: WO 2023/080575

(56) References cited:
- CN-A- 106 137 969
- JP-A- 2017 522 297
- KR-A- 20030 078 722
- KR-A- 20120 053 052
- KR-A- 20200 014 279
- US-A1- 2019 247 357
- US-B2- 8 853 260

## Description

### Technical Field

The present disclosure relates to a method for manufacturing nanoparticles containing taxane and albumin.

The present disclosure also relates to a nanoparticle containing taxane and albumin.

The present disclosure also relates to a method for stabilizing the particle size distribution of nanoparticles containing taxane and albumin.

### Background Art

Poorly soluble drugs refer to drugs that are difficult to dissolve in water due to the compound structure including a hydrophobic moiety therein, and are restrained from application to practical use due to their poor solubility. For instance, over 41% of drugs developed as new pharmaceuticals are abandoned midway due to poor solubility, with more than a third of the drugs listed in the US Pharmacopeia classified as poorly soluble drugs. The use of such poorly soluble drugs requires the addition of extra substances to solve the solubility issue. However, the toxicity of these additional substances often limits their use. For instance, emulsifiers for emulsification or liposomes for encapsulation are commonly used to solubilize poorly soluble substances in water, but these methods are restricted due to the introduction of foreign substances not derived from the human body and physical instability.

Paclitaxel (PTX) is a drug containing a diterpenoid derivative extracted from the bark of Taxus brevifolia and has an alkaloid structure composed of a taxane ring and an ester side chain. It is known that paclitaxel has anti-mitotic activity by promoting the conversion of a tubulin assembly into stable microtubules and is effective against various cancers such as lung cancer, breast cancer, etc. Despite its excellent potential as an anticancer drug, the widespread use of paclitaxel in cancer treatment has been limited due to the low solubility thereof in water. To address the poor solubility thereof, paclitaxel has been previously dissolved in ethanol, but recently, to improve delivery efficiency, paclitaxel is conjugated with albumin and used in injections. Also, it is known that Cremophor EL, a mixture of polyoxyethylated castor oil and absolute ethanol, is used as a solvent to improve paclitaxel's solubility for systemic administration. A commercial formulation using this approach is Taxol^{™}. However, clinically, the excessive administration of such solvents has been reported to cause side effects such as cardiotoxicity, neurotoxicity, neurological disorders, and hypersensitivity.

Abraxane^{™} is a formulation of human serum albumin-bound paclitaxel in a nanoparticle form, developed to overcome the drawbacks of Taxol, such as side effects caused by the solvents. It has shown improved tumor response in human and animal studies and has been successfully used as an anticancer therapeutic to date. However, the manufacturing method for Abraxane, disclosed in patent literature 1 (PCT/US1998/013272), requires immediate rapid depressurization drying after high-pressure homogenization of a mixture of paclitaxel and albumin. If rapid depressurization drying does not immediately follow high-pressure homogenization, the particle size of the nanoparticles containing paclitaxel and albumin significantly increases over time. Thus, if the particle size distribution of nanoparticles does not remain uniform and increases rapidly after high-pressure homogenization, a complex continuous process must be adopted, disadvantaging scale-up, and rapid depressurization drying must be performed, with additional processes needed to remove agglomerated nanoparticles that become unusable as drugs. Moreover, since the starting materials, paclitaxel, and albumin solution concentrations are dilute, at 7.5%(w/v) and 3%(w/v), respectively, according to Example 5 in Patent Literature 1, equipment such as mixing vessels must be large if scaled up, and a concentration process is necessarily required to reduce the volume of the final dispersion, resulting in significantly reduced nanoparticle production.

Throughout this specification, several papers and patent documents are referred to and cited. Further nanoparticles comprising a taxane and an albumin are disclosed in US 2019/247357 A1 and US 8 853 260 B2.

### Disclosure of Invention

### Technical Problem

Intensive and thorough research conducted by the present inventors with the aim of deriving a method for manufacturing albumin-bound taxane nanoparticles that overcomes the problems encountered in the processes disclosed in patent document 1, resulted in the finding that the method for manufacturing nanoparticles containing taxane and albumin according to an aspect of the present disclosure resolves the issue of rapid size increase of nanoparticles after high-pressure homogenization and before or during depressurization drying. Furthermore, it has been proven that the manufacturing method of the present disclosure is suitable for scale-up, providing an environment that is economically and efficiently viable, leading to the present disclosure.

### Summary of the Invention

The invention provides a method for manufacturing nanoparticles containing taxane and albumin, the method comprising the steps of: (a) preparing a first solution in which taxane is dissolved at a concentration of 55 to 75 %(w/v); (b) preparing a second solution in which albumin is dissolved at a concentration of 15 to 25 % (w/v) ; (c) mixing the first solution and the second solution to prepare a suspension; (d) diluting the suspension with an aqueous solvent in an amount of 3 to 12 times the volume of the suspension; and (e) drying the diluted solution to obtain nanoparticles, wherein the taxane is paclitaxel, docetaxel, or a combination thereof.

The invention further provides a nanoparticle containing taxane and albumin, manufactured by the method of the invention.

The invention also provides a method for stabilizing the particle size distribution of nanoparticles containing taxane and albumin, the method comprising the steps of: (a) preparing a first solution in which taxane is dissolved at a concentration of 55 to 75 % (w/v); (b) preparing a second solution in which albumin is dissolved at a concentration of 15 to 25 %(w/v); (c) mixing the first solution and the second solution to prepare a suspension; (d) diluting the suspension with an aqueous solvent in an amount of 3 to 12 times the volume of the suspension; and (e) drying the diluted solution to obtain nanoparticles, wherein the taxane is paclitaxel, docetaxel, or a combination thereof, and wherein the nanoparticles maintain an average particle size D50 of 100 to 200 nm from before the drying is conducted to after the drying is completed, and wherein D50 means that 50% of the particles are smaller than the average particle.

Other features and advantages of the present disclosure will become apparent from the following detailed description, claims, and drawings.

### Detailed Description

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

The term "about", as used herein, including ranges such as temperature, time, pressure, quantity, concentration, etc., can mean a range or specific number of (+) or (-) 10%, 5%, 4%, 3%, 2%, 1%, or somewhere in between.

As used herein, the term "nanoparticles" refer to particles with a dimension size of less than 1 µm. In cases where the nanoparticles of the present disclosure are intended for intravenous injection, it is preferable for the nanoparticles to have a size of 500 nm or less. Specifically, the nanoparticles are preferably in the size range of 50-500 nm, 50-400 nm, 50-300 nm, 50-200 nm, 50-190 nm, 50-180 nm, 50-170 nm, 50-160 nm, 50-150 nm, 80-500 nm, 80-400 nm, 80-300 nm, 80-200 nm, 80-190 nm, 80-180 nm, 80-170 nm, 80-160 nm, 80-150 nm, 100-500 nm, 100-400 nm, 100-300 nm, 100-200 nm, 100-190 nm, 100-180 nm, 100-170 nm, 100-160 nm, 100-150 nm, 110-500 nm, 110-400 nm, 110-300 nm, 110-200 nm, 110-190 nm, 110-180 nm, 110-170 nm, 110-160 nm, or 110-150 nm.

In a group of particles, the collective particle size can be expressed by the average and/or percentile. For example, D₁₀ means that 10% of the particles are smaller than that size, D₅₀ means that 50% of the particles are smaller, and D₉₀ means that 90% of the particles are smaller. The average size of the particles can be expressed as D₅₀.

According to the invention, the concentration of taxane in the first solution is 55 to 75% (w/v). Optionally, the concentration of taxane in the first solution is 60 to 75% (w/v), 65 to 75% (w/v), 70 to 75% (w/v), 55 to 70% (w/v), 55 to 65% (w/v), 55 to 60% (w/v), 60 to 70% (w/v), 64 to 68% (w/v), or 65 to 67% (w/v).

The solvent of the first solution may be selected from the group consisting of ethanol, methanol, isopropanol, butanol, dimethyl sulfoxide (DMSO), dimethylformamide (DMF), tetrahydrofuran (THF), acetonitrile, dichloromethane, ethyl acetate, hexane, diethyl ether, benzene, chloroform, acetone, and combinations thereof, but is not limited thereto.

The solvent of the first solution may be a mixture of ethanol and chloroform. The volume ratio of ethanol: chloroform may be 1:5 to 1:20, 1:6 to 1:20, 1:7 to 1:20, 1:8 to 1:20, 1:9 to 1:20, 1:10 to 1:20, 1:12 to 1:20, 1:14 to 1:20, 1:15 to 1:20, 1:5 to 1:18, 1:6 to 1:18, 1:7 to 1:18, 1:8 to 1:18, 1:9 to 1:18, 1:10 to 1:18, 1:12 to 1:18, 1:14 to 1:18, 1:15 to 1:18, 1:5 to 1:16, 1:6 to 1:16, 1:7 to 1:16, 1:8 to 1:16, 1:9 to 1:16, 1:10 to 1:16, 1:12 to 1:16, 1:14 to 1:16, 1:15 to 1:16, 1:5 to 1:15, 1:6 to 1:15, 1:7 to 1:15, 1:8 to 1:15, 1:9 to 1:15, 1:10 to 1:15, 1:12 to 1:15, or 1:14 to 1:15, but is not limited thereto.

If the concentration of taxane in the first solution is below the lower limit of the range 55 to 75% (w/v) defined in the present disclosure, the particle size, even though performing a water dilution step, rapidly increases to more than 200 nm before reduced pressure drying, rendering the particles unusable as an active ingredient for injectable formulations.

Furthermore, if the concentration of taxane in the first solution exceeds the upper limit of the range 55 to 75% (w/v) defined in the present disclosure, precipitation of paclitaxel occurs during the nanoparticle manufacturing process, preventing normal operation of the high-pressure homogenizer and increasing the particle size. Thus, at concentrations exceeding 75% (w/v), it is impossible to produce uniform-sized nanoparticles.

According to the invention, the taxane is paclitaxel, docetaxel, or a combination thereof.

According to the invention, the concentration of albumin in the second solution is 15 to 25% (w/v). Optionally the concentration of albumin in the second solution is 18 to 25% (w/v), 22 to 25% (w/v), 15 to 22% (w/v), 15 to 18% (w/v), 18 to 22% (w/v), or 19 to 21% (w/v) .

The solvent of the second solution may be an aqueous solvent selected from the group consisting of water, distilled water, sterile water, phosphate-buffered saline (PBS), methanol, purified water, ethanol, 1-propanol, 2-propanol, 1-pentanol, 2-butoxyethanol, ethylene glycol, acetone, 2-butanone, 4-methyl-2-pentanone, chloroform, and combinations thereof, but is not limited thereto.

When the concentration of albumin in the second solution is below the lower limit of the range 15 to 25% (w/v) defined in the present disclosure, the volume of the dispersion must be excessively increased to manufacture the nanoparticles with the same weight ratio of paclitaxel: albumin as in Abraxane (1:9), which deviates from typical filling volume ranges, leading to the need for a concentration process and making industrial application practically difficult. Also, when the concentration of albumin in the second solution exceeds the upper limit of the range 15 to 25% (w/v) defined in the present disclosure, the particle size, even though performing a water dilution step, rapidly increases to more than 200 nm before reduced pressure drying, rendering the particles unusable as an active ingredient for injectable formulations.

The albumin may be human serum albumin (HSA), bovine serum albumin (BSA), ovalbumin (OVA), or a combination thereof.

The volume ratio of mixing the first and second solutions in step (c) may be 1:20 to 1:45, but is not limited thereto. For example, the volume ratio of mixing the first and second solutions may be 1:20 to 1:43, 1:20 to 1:41, 1:20 to 1:39, 1:20 to 1:37, 1:20 to 1:35, 1:20 to 1:33, 1:20 to 1:31, 1:20 to 1:29, 1:20 to 1:27, 1:20 to 1:25, 1:20 to 1:23, 1:20 to 1:21, 1:22 to 1:45, 1:24 to 1:45, 1:26 to 1:45, 1:28 to 1:45, 1:30 to 1:45, 1:32 to 1:45, 1:34 to 1:45, 1:36 to 1:45, 1:38 to 1:45, 1:40 to 1:45, 1:42 to 1:45, 1:44 to 1:45, 1:22 to 1:40, 1:25 to 1:40, 1:25 to 1:35, 1:25 to 1:30, 1:30 to 1:40, or 1:30 to 1:35.

A person skilled in the art can adopt an appropriate ratio considering the reactivity and/or to manufacture nanoparticles from the final product with the same weight ratio of paclitaxel:albumin as 1:9 in Abraxane^{™}. However, the weight ratio of paclitaxel: albumin is not strictly limited to about 1:9; it may range from 1:7 to 1:11, from 1:8 to 1:10, or from 1:8.5 to 1:9.5.

The preparation of the suspension in step (c) may be achieved through high-pressure homogenization.

The high-pressure homogenization may be performed at a temperature condition of 10 to 40°C, 15 to 40°C, 20 to 40°C, 30 to 40°C, or 35 to 40°C, but with no limitations thereto.

The high-pressure homogenization may be performed at pressures of 10,000 to 30,000 psi, 12,000 to 30,000 psi, 15,000 to 30,000 psi, 16,000 to 30,000 psi, 17,000 to 30,000 psi, 18,000 to 30,000 psi, 10,000 to 25,000 psi, 12,000 to 25,000 psi, 15,000 to 25,000 psi, 16,000 to 25,000 psi, 17,000 to 25,000 psi, 18,000 to 25,000 psi, 10,000 to 20,000 psi, 12,000 to 20,000 psi, 15,000 to 20,000 psi, 16,000 to 20,000 psi, 17,000 to 20,000 psi, or 18,000 to 20,000 psi, but with no limitations thereto, wherein 1000 psi = 6.89 MPa.

The high-pressure homogenization may be performed one or more times, more specifically, 1 to 5 times, 1 to 4 times, or 1 to 3 times, but with no limitations thereto.

According to the invention, the dilution in step (d) is performed with 3 to 12 times the volume of the suspension. Optionally the dilution in step (d) is performed 5 to 12 times, 7 to 12 times, 10 to 12 times, 3 to 10 times, 3 to 7 times, 3 to 5 times, 5 to 10 times, or 5 to 7 times the volume of the suspension.

If the amount of aqueous solvent used for dilution is below the lower limit of the range 3 to 12 times defined in the present disclosure, the particle size, even though performing a water dilution step, rapidly increases to more than 200 nm before reduced pressure drying, rendering the particles unusable as an active ingredient for injectable formulations. The increase in particle size may be attributed to the agglomeration of nanoparticles. Agglomeration of nanoparticles leads to the blockage of blood vessels in the case of intravenous nanoparticles; the agglomeration and stability of nanoparticles can affect the efficacy and safety of pharmaceutical compositions containing nanoparticles.

Additionally, if the amount of aqueous solvent used exceeds the upper limit of the range 3 to 12 times defined in this disclosure, not only does the free fraction of paclitaxel, i.e., the unencapsulated rate, increase (from a 0.72% unencapsulated rate at a 5.3 times dilution with water to a 6.24% unencapsulated rate at a 13 times dilution with water), but using more water than necessary for nanoparticle size stabilization also results in inefficiencies and economic disadvantages in scaling up the process.

The nanoparticles in the diluted solution of step (d) have an average size of 100 to 200 nm, or 110 tc 190 nm.

The method of the invention further includes a step of (e) drying the diluted solution to obtain nanoparticles.

The drying may be reduced pressure drying, freeze-drying, or heat drying, but is not limited thereto.

When the drying is reduced pressure drying, it may be performed using a typical reduced pressure drying method such as using a rotary evaporator, without being limited to specific reduced pressure drying conditions.

The reduced pressure drying may be conducted at a temperature condition ranging from 10 to 70°C, 20 to 70°C, 30 to 70°C, 40 to 70°C, 10 to 60°C, 10 to 50°C, 10 to 40°C, 20 to 60°C, 30 to 50°C, 35 to 45°C, 38 to 42°C, or 39 to 41°C, but with no limitations thereto.

The reduced pressure drying may be conducted at a pressure condition ranging from 10 to 110 hPa, 20 to 110 hPa, 30 to 110 hPa, 40 to 110 hPa, 50 to 110 hPa, 10 to 100 hPa, 10 to 90 hPa, 10 to 80 hPa, 10 to 70 hPa, 20 to 100 hPa, 30 to 90 hPa, 40 to 80 hPa, 50 to 70 hPa, or 55 to 65 hPa, but with no limitations thereto.

The nanoparticles present in the diluted solution can maintain an average size of 100 to 200 nm, preferably 110 to 190 nm, from before the drying is conducted to after the drying is completed.

The method for producing nanoparticles containing taxane and albumin provided in one aspect of the present disclosure demonstrates a remarkable effect of maintaining uniform size and structural stability of the nanoparticles in the diluted solution before reduced pressure drying over time, compared to the conventional process of rapidly reduced pressure drying after high-pressure homogenization without performing a dilution step with water. This effect of the present disclosure is supported by the Examples, Comparative Examples, and Experimental Examples described later.

Provided according to another aspect of the present disclosure are nanoparticles containing paclitaxel and albumin produced by the method according to one aspect of the present disclosure.

Nanoparticles containing paclitaxel and albumin produced by the method described herein, when reconstituted in an aqueous solvent (including *in vivo* and *in vitro*) after being dried, may possess substantially the same therapeutic activity and particle characteristics as the nanoparticles before drying (e.g., nanoparticles in the diluted solution of step (d)).

When the dried nanoparticles are reconstituted in an aqueous solvent, the average size of the particles and the distribution of particle sizes are substantially the same as those of the nanoparticles before drying. The term "substantially the same" means that the parameters for the nanoparticles after freeze-drying fall within ±10%, more specifically within ±7%, ±5%, ±4%, or ±3.5% range, compared to the parameters for the nanoparticles before freeze-drying.

The dried nanoparticles may be stable at room temperature for at least 3 months, 4 months, 5 months, 6 months, 7 months, 8 months, 9 months, 10 months, 11 months, 12 months, or longer, maintaining their therapeutic activity and particle characteristics even after being stored at room temperature for said period and then reconstituted in an aqueous solvent.

The drying may be freeze-drying.

Freeze-drying means a process of freezing the nanoparticles, which are the material to be dried, and then removing the frozen solvent by sublimation in a vacuum environment. The freeze-drying process may optionally use an excipient or cryoprotectant to enhance the storage stability of the freeze-dried product.

Examples of the excipient or cryoprotectant include polymers, such as dextran and polyethylene glycol; sugars, such as sucrose, glucose, trehalose, and lactose; surfactants, such as polysorbates; and amino acids, such as glycine, arginine, and serine, but are not limited thereto.

The nanoparticles may further include one or more additional therapeutic agents in addition to taxane of the present disclosure.

The nanoparticles of the present disclosure may be pharmaceutically formulated for administration to a patient. Various formulations and drug delivery systems are available in the art. For example, reference can be made to Gennaro, A.R., ed. (1995) Remington's Pharmaceutical Sciences, 18th ed., Mack Publishing Co.

The nanoparticles of the present disclosure may be administered as a pharmaceutical composition via one of the following routes: oral; non-oral, for example, transdermal, intranasal, intramuscular, intravenous, subcutaneous, intradermal, intra-tumoral, etc.

The nanoparticles may include solid, liquid, semi-solid, or gaseous excipients.

Examples of the solid pharmaceutical excipient include starch, cellulose, talc, glucose, lactose, sucrose, gelatin, malt, rice, flour, silica gel, magnesium stearate, sodium stearate, glyceryl monostearate, sodium chloride, dried skim milk, and the like.

The liquid and semi-solid excipients may be selected from various oils including glycerol, propylene glycol, water, ethanol and petroleum, and oils of animal, vegetable, or synthetic origin, for example, peanut oil, soybean oil, mineral oil, sesame oil, etc. Preferred liquid carriers, especially for injectable solutions, include water, saline, aqueous dextrose, and glycols. For other suitable pharmaceutical excipients and their formulations, reference may be made to Remington's Pharmaceutical Sciences (Mack Publishing Company, 18th ed., 1990) edited by E. W. Martin.

The nanoparticles may be formulated for intravenous injection. The nanoparticles may be formulated for direct injection or perfusion into a tumor.

The invention provides a method for stabilizing the particle size distribution of nanoparticles, the method comprising the steps of:
(a) preparing a first solution in which paclitaxel is dissolved at a concentration of 55 to 75 %(w/v);
(b) preparing a second solution in which albumin is dissolved at a concentration of 15 to 25 %(w/v);
(c) mixing the first solution and the second solution to prepare a suspension;
(d) diluting the suspension with an aqueous solvent in an amount of 3 to 12 times the volume of the suspension; and
(e) drying the diluted solution to obtain nanoparticles,
   wherein the taxane is paclitaxel, docetaxel, or a combination thereof, and
   wherein the nanoparticles maintain an average particle size D50 of 100 to 200 nm from before the drying is conducted to after the drying is completed, and wherein D50 means that 50% of the particles are smaller than the average particle size.

Since the method for stabilizing the particle size distribution of nanoparticles according to one aspect of the present disclosure includes the same steps (a) to (e) as the method for producing nanoparticles according to an aspect of the present disclosure, the common contents therebetween are omitted to avoid redundancy.

### Advantageous Effects of Invention

According to the disclosure, the method for producing nanoparticles containing paclitaxel and albumin according to the present disclosure can be usefully employed as an improved method for producing nanoparticles containing paclitaxel and albumin, as compared to conventional processes for producing nanoparticles since the average size of the nanoparticles produced by the method according to the present disclosure remains uniformly maintained over time, resulting in excellent structural stability and particle size distribution.

### Best Mode for Carrying out the Invention

A better understanding of the present disclosure may be obtained through the following Examples, which are set forth to illustrate, but are not to be construed to limit the present disclosure.

### EXAMPLES

Unless otherwise stated, "%" used to indicate the concentration of a specific substance is (weight/weight) % for solid/solid, (weight/volume) % for solid/liquid, and (volume/volume) % for liquid/liquid throughout the specification.

### <COMPARATIVE EXAMPLE 1>

### Manufacture of Nanoparticles Containing Paclitaxel and Albumin According to Conventional Method 1

Nanoparticles containing paclitaxel and albumin were prepared according to the method of Example 4 in related patent literature 1 (PCT/US1998/013272). Briefly, 30 mg of paclitaxel was dissolved in a mixture of 0.55 ml of chloroform and 0.05 ml of ethanol (11:1 v/v) to prepare a 5% (w/v) concentration of paclitaxel solution (first solution). Also, 29.4 ml of a 1% (w/v) human serum albumin (HSA) solution pre-saturated with 1% chloroform was prepared (second solution). The first and the second solution were mixed and homogenized using an IKA homogenizer (T-25) at 5,600 rpm for 5 minutes to form a coarse emulsion which was then transferred to a Micronox (MN400BF) and subjected to high-temperature homogenization under conditions of 22°C and 18,000 psi for 2 cycles. After completion of the high-pressure homogenization, the homogenate was transferred to a rotary evaporator, and the chloroform was quickly removed under reduced pressure conditions of 40°C, 60 hPa for 15 to 30 minutes to afford nanoparticles containing paclitaxel and albumin.

### COMPARATIVE EXAMPLE 2

### Manufacture of Nanoparticles Containing Paclitaxel and Albumin According to Conventional Method 2

Nanoparticles containing paclitaxel and albumin were manufactured according to the method of Example 5 in related patent literature 1 (PCT/US1998/013272). Briefly, 225 mg of paclitaxel was dissolved in a mixture of 2.7 ml of chloroform and 0.3 ml of ethanol (9:1 v/v) to prepare a 7.5% (w/v) concentration of paclitaxel solution (first solution). Also, 97 ml of a 3% (w/v) human serum albumin (HSA) solution (second solution) was prepared. The first and the second solution were mixed and homogenized using an IKA homogenizer (T-25) at 5,600 rpm for 5 minutes to form a coarse emulsion which was then transferred to a Micronox (MN400BF) and subjected to high-pressure homogenization under conditions of 22°C and 18,000 psi for 2 cycles. The high-pressure homogenized mixture was then transferred to a rotary evaporator, and the chloroform was quickly removed under reduced pressure conditions of 40°C, 60 hPa for 15 to 30 minutes to afford nanoparticles containing paclitaxel and albumin.

### EXAMPLES 1 TO 14

### Manufacture of Nanoparticles Containing Paclitaxel and Albumin According to the Present Disclosure

Nanoparticles containing paclitaxel and albumin were manufactured as follows. The experimental protocol below corresponds to Example 3 in Table 1 below.

In 1.5 ml of a mixture of chloroform/ethanol (14:1 v/v) was dissolved 1 g of paclitaxel to prepare a 66.7% (w/v) concentration of paclitaxel solution (first solution). Also, 45 ml of a 20% (w/v) concentration of albumin solution (second solution, Greencross Human Serum Albumin Inj. 20%) was prepared. The first and the second solution were mixed and homogenized using an IKA homogenizer (T-25) at 5,600 rpm for 5 minutes. Subsequently, the mixture was transferred to a Micronox (MN400BF) and subjected to high-pressure homogenization under conditions of 22°C and 18,000 psi for 2 cycles. After completion of the high-pressure homogenization, the homogenate was diluted with water at a 5.3-fold volume ratio. The diluted mixture was dried under the condition of 40°C and 60 hPa using a rotary evaporator to afford nanoparticles containing paclitaxel and albumin.

Nanoparticles were manufactured in the same manner as the above-described method, with the exception of various changes in the concentration of the first and the second solution or the amount of water used for dilution, and the concrete variable conditions for each example are listed in Table 1 below.

In Table 1, the mixing volume ratio of the first and the second solution was determined based on the concentrations of the first and the second solutions to manufacture the final nanoparticles with a paclitaxel:albumin weight ratio of 1:9 as that in Abraxane^{®}.

**TABLE 1**

| Ex. # | 1^{st} Sol'n Conc. % (w/v) | 2^{nd} Sol' n Conc. % (w/v) | Water amount (fold) | Mixing volume ratio of 1^{st} and 2^{nd} Sol' n |
|---|---|---|---|---|
| 1 | 55.0 | 20 | 5.3 | 1 : 24.8 |
| 2 | 60.0 | | | 1 : 27.0 |
| 3 | 66.7 | | | 1 : 30.0 |
| 4 | 70.0 | | | 1 : 31.5 |
| 5 | 75.0 | | | 1 : 33.8 |
| 6 | 66.7 | 15 | 5.3 | 1 : 40.0 |
| 7 | | 18 | | 1 : 33.4 |
| 8 | | 22 | | 1 : 27.3 |
| 9 | | 25 | | 1 : 24.0 |
| 10 | 66.7 | 20 | 3.0 | 1 : 30.0 |
| 11 | | | 5.0 | |
| 12 | | | 7.0 | |
| 13 | | | 10.0 | |
| 14 | | | 12.0 | |
| C. Ex. 1 | 5 | 1 | - | 1 : 49.0 |
| C. Ex. 2 | 7.5 | 3 | - | 1 : 32.3 |

The manufacturing methods of Examples 1 to 14 described in Table 1 differ from those described in Examples 4 and 5 of related patent literature 1 (PCT/US1998/013272) (Comparative Examples 1 and 2) in terms of i) the concentration of the first and second solutions and ii) whether a process of diluting with water is additionally included before the process of drying the homogenate under reduced pressure.

### COMPARATIVE EXAMPLES 3 TO 12

Manufacture of Nanoparticles Containing Paclitaxel and Albumin Without Dilution Step

Experiments in Comparative Examples 3 to 12 were conducted so as to examine how the manufacturing methods of Examples 1 to 14, which further include a process of diluting the homogenate with water before the process of drying under reduced pressure, compared to those of Comparative Examples 1 and 2, affect the quality of the manufactured nanoparticles containing paclitaxel and albumin.

Briefly, nanoparticles containing paclitaxel and albumin were manufactured in the same manner as in Examples 1 to 14, with the exception of further conducting a step of diluting with water after mixing of the first and the second solution and homogenization at a high pressure and before drying at a reduced pressure. Specific variable conditions for each Comparative Example are listed in Table 2 below.

**TABLE 2**

| C. Ex. # | 1^{st} Sol'n Conc. % (w/v) | 2^{nd} Sol' n Conc. % (w/v) | Water amount (fold) | Mixing volume ratio of 1^{st} and 2^{nd} Sol'n |
|---|---|---|---|---|
| 3 | 55.0 | 20 | | 1 : 24.8 |
| 4 | 60.0 | | | 1 : 27.0 |
| 5 | 66.7 | | | 1 : 30.0 |
| 6 | 70.0 | | X (only water dilution step not conducted) | 1 : 31.5 |
| 7 | 75.0 | | | 1 : 33.8 |
| 8 | 66.7 | 15 | | 1 : 40.0 |
| 9 | | 18 | | 1 : 33.4 |
| 10 | | 22 | | 1 : 27.3 |
| 11 | | 25 | | 1 : 24.0 |
| 12 | 66.7 | 20 | | 1 : 30.0 |

### COMPARATIVE EXAMPLES 13 to 24

Manufacture of nanoparticles containing paclitaxel and albumin Based on i) concentration of first solution (%(w/v)), ii) concentration of second solution (%(w/v)), and iii) amount of water used for dilution

The experiments of Comparative Examples 13 to 24 were conducted to examine the impact of i) concentration of the first solution (%(w/v)), ii) concentration of the second solution (%(w/v)), and iii) amount of water used for dilution, set in Examples 1 to 14 of the present disclosure, on the quality of the manufactured nanoparticles containing paclitaxel and albumin.

Briefly, nanoparticles containing paclitaxel and albumin were manufactured in the same manners as in Examples 1 to 14, with the exception of using conditions that deviate from the ranges of the concentration of the first solution (%(w/v)), the concentration of the second solution (%(w/v)), and the amount of water used for dilution. The concrete variable conditions for each Comparative Example are shown in Table 3 below.

Similarly to Examples 1 to 14, the volume mixing ratios of the first and second solutions were determined based on the concentrations of the first and second solutions, as shown in Table 3, below, to manufacture the final nanoparticles with a paclitaxel:albumin weight ratio of 1:9 as in Abraxane^{®}.

**TABLE 3**

| C. Ex. # | 1^{st} Sol'n Conc. % (w/v) | 2^{nd} Sol'n Conc. % (w/v) | Water amount (fold) | Mixing volume ratio of 1st and 2^{nd} Sol'n |
|---|---|---|---|---|
| 13 | 45.0 | 20 | 5.3 | 1 : 20.3 |
| 14 | 50.0 | | | 1: 22.5 |
| 15 | 80.0 | | | 1 : 36.0 |
| 16 | 85.0 | | | 1 : 38.3 |
| 17 | 66.7 | 5 | 5.3 | 1 : 120.0 |
| 18 | | 10 | | 1 : 60.0 |
| 19 | | 30 | | 1: 20.0 |
| 20 | | 35 | | 1 : 17.1 |
| 21 | 66.7 | 20 | 1.0 | 1 : 30.0 |
| 22 | | | 2.0 | |
| 23 | | | 13.0 | |
| 24 | | | 14.0 | |

### EXPERIMENTAL EXAMPLE 1

Comparative Evaluation for Superiority in Particle Size Distribution Maintenance between Inventive Nanoparticles and Conventional Process Nanoparticles

### (PCT/US1998/013272)

When the particle size distribution (PSD) of the nanoparticles within the homogenate remains consistent until the solvent is completely removed during the reduced pressure drying step after high-pressure homogenization, nanoparticles containing paclitaxel and albumin can be manufactured at a high yield.

Furthermore, if the effect of maintaining a consistent particle size distribution of the nanoparticles for a certain period of time before reduced pressure drying and after high-pressure homogenization is achieved, the stability in the particle size of the nanoparticle products during the manufacturing process is secured, making the process actively usable as a technology suitable for economic and efficient upscaling.

Hence, comparative evaluation was made to examine whether the present disclosure's manufacturing methods of Examples 1 to 14 and the conventional methods of Comparative Examples 1 and 2 were comparatively evaluated to determine if the particle size distribution of the nanoparticles manufactured by the conventional methods of Comparative Examples 1 and 2 and the methods of Examples 1 to 14 of the present disclosure remained stable over time within the mixture before reduced pressure drying (in the case of Examples, within dilutions in water).

In brief, using a Zetasizer (Malvern Zetasizer, Malvern Instruments Ltd.), the particle size distributions of the nanoparticles within the homogenate from Comparative Examples 1 to 2, and the mixture diluted with water from Examples 1 to 14 were evaluated over time using Malvern Zetasizer (Malvern Instruments Ltd.). The measurement method of the suspension in the Zetasizer included adjusting the following parameters: temperature 25.0°C, scattering angle 90°, refractive index dispersion 1.33, viscosity (automatic) 0.8872cP. A dilution of 1 ml of each resuspended suspension in 10 ml of triple-distilled water was transferred in an amount of 1 ml to each cuvette and placed in the cuvette holder of the Zetasizer before starting the particle size analysis. The average values were obtained after repeating the analysis 3 times through multimodal analysis.

The results are summarized in Table 4 below.

**TABLE 4**

| Ex. | Z-average (nm) | | | | | | |
|---|---|---|---|---|---|---|---|
| | 0 min | 20 min | 40 min | 60 min | 80 min | 100 min | 120 min |
| 1 | 146.5 | 156.8 | 157.8 | 158.9 | 157.5 | 159.3 | 160.2 |
| 2 | 151.1 | 151.2 | 151.8 | 152.3 | 151.7 | 152.8 | 151.8 |
| 3 | 155.3 | 156.4 | 154.8 | 155.2 | 155.3 | 154.8 | 154.6 |
| 4 | 156.7 | 156.2 | 155.8 | 157.8 | 156.8 | 157.2 | 158.4 |
| 5 | 160.6 | 159.8 | 160.1 | 160.8 | 161.2 | 160.8 | 159.8 |
| 6 | 153.5 | 153.2 | 153.9 | 154.2 | 154.8 | 153.2 | 153.8 |
| 7 | 154.1 | 153.8 | 154.5 | 156.5 | 155.8 | 154.2 | 155.7 |
| 8 | 153.3 | 153.1 | 152.8 | 154.7 | 154.2 | 154.7 | 154.3 |
| 9 | 153.3 | 161.2 | 163.5 | 164.2 | 163.2 | 164.2 | 163.2 |
| 10 | 150.3 | 175.4 | 178.6 | 179.2 | 178.6 | 179.5 | 178.8 |
| 11 | 155.2 | 154.8 | 154.9 | 155.4 | 155.8 | 154.1 | 153.7 |
| 12 | 153.6 | 154.8 | 153.4 | 155.2 | 153.8 | 154.2 | 152.6 |
| 13 | 149.8 | 150.2 | 150.8 | 151.4 | 150.8 | 151.8 | 152.8 |
| 14 | 151.3 | 151.8 | 150.9 | 150.2 | 150.6 | 152.8 | 153.7 |
| C. Ex. 1 | 151.3 | 430.1 | 472.7 | 562.8 | 696.1 | 798.5 | 805.3 |
| C. Ex. 2 | 158.2 | 458.6 | 508.4 | 578.3 | 689.4 | 781.6 | 813.6 |

As shown in Table 4, the manufacturing method of the present disclosure (Examples 1 to 14), which includes a step of diluting with water before reduced pressure drying and after high-pressure homogenization, maintained the size of nanoparticles over time, demonstrating that the structural stability and particle size distribution of nanoparticles are relatively excellent, compared to the conventional manufacturing methods (Comparative Examples 1 and 2) that quickly proceed to reduced pressure drying after high-pressure homogenization without a dilution step.

Specifically, the nanoparticles of Comparative Examples 1 and 2, corresponding to Examples 4 and 5 of PCT/US1998/013272, showed an increase in Z-average (nm) to more than 430.1 nm and 458.6 nm, respectively, just 20 minutes after high-pressure homogenization, indicating that they were no longer usable as active ingredients in injectable forms. In contrast, nanoparticles of all the Examples 1 to 14 of the present disclosure maintained a Z-average (nm) of <200 nm even after 120 minutes, indicating an excellent particle size distribution.

The maintenance of an excellent particle size distribution of nanoparticles in the mixture before reduced pressure drying (in the case of Examples, in dilution in water) offers significant advantages in configuring a upscaling process.

More specifically, if the particle size distribution of nanoparticles does not remain uniform and increases rapidly after high-pressure homogenization, like the process disclosed in PCT/US1998/013272, complex continuous processes must be adopted, which is very disadvantageous for upscaling. The process also needs to proceed quickly to reduced pressure drying, and an additional process of removing agglomerated nanoparticles, which are unusable as drugs, might be required. Moreover, since the starting materials, paclitaxel, and albumin solutions are dilute with respective concentrations of 7.5% (w/v) and 3% (w/v), based on Example 5 of PCT/US1998/013272, the volume of equipment like mixing tanks must be large if upscaling as is, and a concentration process is necessarily performed to reduce the volume of the final dispersion, leading to a significant decrease in nanoparticle production.

On the other hand, if the particle size distribution within the diluted mixture remains uniform due to the dilution step as in the method of <Examples 1-14> according to the present disclosure, there is no need to adopt complex continuous processes, no need to rapidly perform reduced pressure drying, no need for an additional process to remove agglomerated nanoparticles unusable as drugs, and a significant improvement in the final nanoparticle yield occurs. Furthermore, the manufacturing method provided by the present disclosure uses the first and second solutions at high concentrations, reducing the volume of raw materials and thus offering improved workability when considering upscaling.

Therefore, the nanoparticle manufacturing method provided by one aspect of the present disclosure not only resolves the issue of rapid increase in the size of nanoparticles until reduced pressure drying after high-pressure homogenization, as disclosed in PCT/US1998/013272, but also provides an environment suitable for upscaling in terms of economy and efficiency.

### EXPERIMENTAL EXAMPLE 2

Comparative Evaluation of Nanoparticles for Maintenance of Particle Size Distribution Whether or not to Conduct Water Dilution Step

Examples 1 to 14 differ from Comparative Examples 3 to 12 in terms of whether the water dilution step is performed or omitted, while all other conditions remain the same.

Using the same method as in Experimental Example 1, comparative evaluation was made to examine whether the particle size distribution of nanoparticles in the mixture before reduced pressure drying (in dilution in water for the Examples) manufactured in Examples 1 to 14 and Comparative Examples 3 to 12 remained stable over time.

The results are presented in Table 5.

**TABLE 5**

| Ex. | Z-average (nm) | | | C. Ex. | Z-average (nm) | | |
|---|---|---|---|---|---|---|---|
| | 0 min | 60 min | 120 min | | 0 min | 60 min | 120 min |
| 1 | 146.5 | 158.9 | 160.2 | 3 | 142.2 | 601.6 | 687.3 |
| 2 | 151.1 | 152.3 | 151.8 | 4 | 152.6 | 586.9 | 651.2 |
| 3 | 155.3 | 155.2 | 154.6 | 5 | 154.8 | 526.2 | 631.9 |
| 4 | 156.7 | 157.8 | 158.4 | 6 | 154.2 | 523.1 | 625.1 |
| 5 | 160.6 | 160.8 | 159.8 | 7 | 157.9 | 503.8 | 594.2 |
| 6 | 153.5 | 154.2 | 153.8 | 8 | 152.1 | 581.8 | 648.3 |
| 7 | 154.1 | 156.5 | 155.7 | 9 | 155.8 | 572.9 | 637.2 |
| 8 | 153.3 | 154.7 | 154.3 | 10 | 154.2 | 598.8 | 647.3 |
| 9 | 153.3 | 164.2 | 163.2 | 11 | 155.8 | 618.9 | 662.1 |
| 10 | 150.3 | 179.2 | 178.8 | 12 | 154.8 | 526.4 | 631.9 |
| 11 | 155.2 | 155.4 | 153.7 | | | | |
| 12 | 153.6 | 155.2 | 152.6 | | | | |
| 13 | 149.8 | 151.4 | 152.8 | | | | |
| 14 | 151.3 | 150.2 | 153.7 | | | | |

As shown in Table 5, the nanoparticles within the diluted mixture of Examples 1 to 14 of the present disclosure, which underwent a water dilution step after high-pressure homogenization and before reduced pressure drying, maintained a uniform particle size distribution over time. In contrast, the nanoparticles in the mixture of Comparative Examples 3 to 12, which did not include a water dilution step, grew rapidly over time to a size that rendered them unusable as active ingredients in injectable forms.

From these results, it can be seen that performing the water dilution step included in the examples of the present disclosure is essential to secure the advantages described in Experimental Example 1 when configuring an upscaling process.

### EXPERIMENTAL EXAMPLE 3

Comparative Evaluation of Nanoparticles for Maintenance of Particle Size Distribution Based on i) Concentration of Solution 1 (%(w/v)), ii) Concentration of Solution 2 (%(w/v)), and iii) Amount of Water Used for Dilution

To examine the effects of i) the concentration of solution 1 (%(w/v)), ii) the concentration of solution 2 (%(w/v)), and iii) the amount of water used for dilution on the particle size distribution of the nanoparticles of the present disclosure, nanoparticles were manufactured according to Comparative Examples 13 to 24 and measured for particle size distribution using the same method as in Experimental Example 1.

### Experimental Example 3-1. Concentration of solution 1 (%(w/v))

The manufacturing conditions for Comparative Examples 13 to 16 are shown in Table 6, and the particle size distribution data is presented in Table 7.

**TABLE 6**

| C. Ex. | 1^{st} Sol'n Conc. % (w/v) | 2^{nd} Sol'n Conc. % (w/v) | Water amount (fold) | Mixing volume ratio of 1st and 2^{nd} Sol'n |
|---|---|---|---|---|
| 13 | 45.0 | | | 1 : 20.3 |
| 14 | 50.0 | 20 | 5.3 | 1 : 22.5 |
| 15 | 80.0 | | | 1 : 36.0 |
| 16 | 85.0 | | | 1 : 38.3 |

**TABLE 7**

| C. Ex. | Z-average (nm) | | | | | | |
|---|---|---|---|---|---|---|---|
| | 0 min | 20 min | 40 min | 60 min | 80 min | 100 min | 120 min |
| 13 | 149.9 | 189.5 | 205.6 | 227.6 | 231.5 | 236.6 | 231.5 |
| 14 | 148.4 | 180.9 | 196.8 | 218.6 | 218.1 | 220.8 | 225.9 |
| 15 | - | - | - | - | - | - | - |
| 16 | - | - | - | - | - | - | - |

| | | | | | | | |
|---|---|---|---|---|---|---|---|
| -: In the nanoparticle manufacturing process, precipitation of paclitaxel occurred, making it impossible for the high-pressure homogenizer to operate normally, and the particle size of the nanoparticles increased to a level that could not be measured. | | | | | | | |

As indicated in Table 7, when the concentration of the first solution was below the lower limit of the range 55-75% (w/v) defined in this disclosure (i.e., in Comparative Examples 13 and 14), even after performing the water dilution step, the particle size increased rapidly to over 200 nm before reduced-pressure drying, making the particles unsuitable for use as an active ingredient in injectable form.

Furthermore, when the concentration of Solution 1 exceeded the upper limit of the defined range 55-75% (w/v) (i.e., in Comparative Examples 15 and 16), paclitaxel precipitated during the manufacturing process, making it impossible for the high-pressure homogenizer to operate normally. It was also found that it was not possible to produce nanoparticles with a uniform size when the concentration of Solution 1 exceeded 75% (w/v).

### Experimental Example 3-2. Concentration of solution 2 (%(w/v))

The manufacturing conditions for Comparative Examples 17 to 20 are shown in Table 8, and the particle size distribution data is presented in Table 9.

**TABLE 8**

| C. Ex. | 1^{st} Sol'n Conc. % (w/v) | 2^{nd} Sol'n Conc. % (w/v) | Water amount (fold) | Mixing volume ratio of 1st and 2^{nd} Sol'n |
|---|---|---|---|---|
| 17 | | 5 | | 1 : 120.0 |
| 18 | 66.7 | 10 | 5.3 | 1 : 60.0 |
| 19 | | 30 | | 1: 20.0 |
| 20 | | 35 | | 1 : 17.1 |

**TABLE 9**

| C. Ex. | Z-average (nm) | | | | | | |
|---|---|---|---|---|---|---|---|
| | 0 min | 20 min | 40 min | 60 min | 80 min | 100 min | 120 min |
| 19 | 166.4 | 204.2 | 216.8 | 226.5 | 221.5 | 228.8 | 226.7 |
| 20 | 167.8 | 216.8 | 226.1 | 239.1 | 240.5 | 241.6 | 245.8 |

With reference to Tables 8 and 9, when the concentration of Solution 2 was below the lower limit of the defined range 15-25% (w/v) (i.e., as in Comparative Examples 17 and 18), the volume of the dispersion needed to achieve a paclitaxel:albumin weight ratio of 1:9, identical to that of AbraxaneTM, increased excessively, as exemplified by1 : 120.0 and 1 : 60.0 for the first and the second mixing volume ratios in Table 8 . This resulted in a problem where the volume ratio of the mixture exceeded the typical filling volume range, necessitating an additional concentration process and making industrial application practically difficult.

Additionally, when the concentration of Solution 2 exceeded the upper limit of the defined range 15-25% (w/v) (i.e., as in Comparative Examples 19 and 20), it was found that even performing the water dilution step could not prevent the particle size from rapidly increasing to over 200 nm before reduced pressure drying, rendering them unusable as active ingredients in injectable forms.

### Experimental Example 3-3. Amount of water

The manufacturing conditions for Comparative Examples 21 to 24 are presented in Table 10, and the particle size distribution data is shown in Table 11.

**TABLE 10**

| C. Ex. | 1^{st} Sol' n Conc. % (w/v) | 2^{nd} Sol'n Conc. % (w/v) | Water amount (fold) | Mixing volume ratio of 1st and 2^{nd} Sol'n |
|---|---|---|---|---|
| 21 | | | 1.0 | |
| 22 | 66.7 | 20 | 2.0 | 1 : 30.0 |
| 23 | | | 13.0 | |
| 24 | | | 14.0 | |

**TABLE 11**

| C. Ex. | Z-average (nm) | | | | | | |
|---|---|---|---|---|---|---|---|
| | 0 min | 20 min | 40 min | 60 min | 80 min | 100 min | 120 min |
| 21 | 153.8 | 242.5 | 268.8 | 278.5 | 271.5 | 289.5 | 281.5 |
| 22 | 154.2 | 202.7 | 223.8 | 235.8 | 234.6 | 239.5 | 248.1 |

As indicated in Table 11, when the amount of water used for dilution was below the lower limit of the defined range of 3-12 times (i.e., as in Comparative Examples 21, 22), the particle size rapidly increased to over 200 nm before reduced pressure drying, rendering them unusable as active ingredients for injections, even after performing the water dilution step.

Additionally, when the amount of water used exceeded the upper limit of the defined range of 3-12 times (i.e., as in Comparative Examples 23, 24), not only did the free fraction of paclitaxel, i.e., the unencapsulated rate, increase significantly (from 0.72% at 5.3 times dilution to 6.24% at 13 times dilution), but the use of more water than necessary for nanoparticle size stabilization also led to inefficient and uneconomical issues in the upscaling process.

The unencapsulated rate of paclitaxel was determined as follows:
The specimens manufactured under the conditions of Example 3 (5.3 times water dilution) and Comparative Example 23 (13 times water dilution) were ultrafiltered and the filtrates were analyzed using HPLC.

### <HPLC analysis of unencapsulated paclitaxel>

Chromatography system
HPLC: Agilent Technologies G7111A solvent delivery system
Agilent Technologies G7129A autoinjector
Agilent Technologies G7114A variable wavelength detector
Agilent Technologies G7116A column oven
Column: FluoroSep-RP Phenyl, 5 µm, 4.6 mm x 25 cm, Perkin Elmer^{®}
Column Temp.: 25°C
Mobile phase: water/acetonitrile 11:9
Flow rate: 1.5mL/min
Detection: 227 nm
Injection amount: 10 µL
Analysis time: 20 min

As a result, when diluted with 5.3 times the amount of water as in Example 3, the non-incorporation rate of paclitaxel was 0.72%, and when diluted with 13 times the amount of water as in Comparative Example 23, the non-encapsulation rate of paclitaxel was 6.24%. The difference in manufacturing methods between Example 3 and Comparative Example 23 was solely in the amount of water used for dilution, highlighting the importance of water dilution quantity in introducing a upscaling process for manufacturing high-quality nanoparticles.

From the results of Experimental Examples 2 and 3, it is evident that the nanoparticle manufacturing method according to Examples 1 to 14 of the present disclosure, where the concentration of Solution 1 and 2 and the water dilution step before reduced pressure drying are integrally linked, achieves the significant effect of maintaining a consistent particle size distribution within the diluted suspension after high-pressure homogenization. This leads to the manufacture of nanoparticles containing paclitaxel and albumin at a high production rate.

Moreover, as the present disclosure demonstrates that the particle size distribution remains consistent even after some time has passed following high-pressure homogenization, it overcomes the limitations of conventional processes, such as the need for complex continuous processes and additional steps to remove agglomerated nanoparticles unusable as drugs. This makes the technology economically viable, efficient, and suitable for upscaling, thereby enabling its active utilization.

### EXPERIMENTAL EXAMPLE 4

Evaluation of Particle Size Distribution Before and After Freeze-Drying and Rehydration (Uniformity and Ease of Regeneration of Particle Size)

Following the manufacturing process of paclitaxel and albumin-containing nanoparticles as described in Examples 1 to 14, examination was conducted to assess whether the particle size remains uniform i) before freeze-drying and ii) upon rehydration after freeze-drying.

More specifically, the dispersion with organic solvent removed via reduced pressure drying was filtered through a 0.22 µm pore size filter (Sartorius). The filtered liquid was then filled into glass vials for freeze-drying, which was carried out for 72 hours. After freeze-drying, 20 ml of tertiary water or 0.9% saline was added to the vials containing the freeze-dried powder to re-disperse them. The results of the particle size distribution measurements are presented in Table 12.

**TABLE 12**

| Ex. | Z-average (nm) | |
|---|---|---|
| | Before free-drying | Rehydration after free drying |
| 1 | 146.5 | 151.6 |
| 2 | 151.1 | 150.9 |
| 3 | 155.3 | 154.2 |
| 4 | 156.7 | 155.4 |
| 5 | 160.6 | 159.3 |
| 6 | 153.5 | 153.6 |
| 7 | 154.1 | 154.8 |
| 8 | 153.3 | 153.9 |
| 9 | 153.3 | 154.8 |
| 10 | 150.3 | 151.4 |
| 11 | 155.2 | 154.7 |
| 12 | 153.6 | 153.0 |
| 13 | 149.8 | 150.4 |
| 14 | 151.3 | 151.6 |

As indicated in Table 12, the nanoparticles manufactured according to Examples 1 to 14 of the present disclosure maintained a uniform particle size before freeze-drying and after rehydration following freeze-drying.

## Claims

1. A method for manufacturing nanoparticles containing taxane and albumin, the method comprising the steps of:
(a) preparing a first solution in which taxane is dissolved at a concentration of 55 to 75 %(w/v);
(b) preparing a second solution in which albumin is dissolved at a concentration of 15 to 25 %(w/v);
(c) mixing the first solution and the second solution to prepare a suspension;
(d) diluting the suspension with an aqueous solvent in an amount of 3 to 12 times the volume of the suspension; and
(e) drying the diluted solution to obtain nanoparticles,
wherein the taxane is paclitaxel, docetaxel, or a combination thereof.

2. The method of claim 1, wherein the nanoparticles in the diluted solution maintain an average size D₅₀ of 100 to 200 nm from before the drying is conducted to after the drying is completed, and wherein D₅₀ means that 50% of the particles are smaller than the average particle size.

3. The method of claim 1, wherein the nanoparticles in the diluted solution maintain an average size D₅₀ of 110 to 190 nm from before the drying is conducted to after the drying is completed, and wherein D₅₀ means that 50% of the particles are smaller than the average particle size.

4. The method of claim 1, wherein the albumin is human serum albumin (HSA), bovine serum albumin (BSA), ovalbumin (OVA), or a combination thereof.

5. The method of claim 1, wherein the first solution and second solutions in step (c) are mixed at a volume ratio of 1:20 to 1:45.

6. The method of claim 1, wherein the suspension in step (c) is prepared by high-pressure homogenization.

7. The method of claim 6, wherein the high-pressure homogenization is carried out in a temperature condition of 10 to 40°C.

8. The method of claim 6, wherein the high-pressure homogenization is carried out in a pressure condition of 68.95 MPa to 206.84 MPa (10000 to 30000 psi).

9. A nanoparticle containing taxane and albumin, manufactured by the method of any one of claims 1 to 8.

10. A method for stabilizing the particle size distribution of nanoparticles containing taxane and albumin, the method comprising the steps of:
(a) preparing a first solution in which taxane is dissolved at a concentration of 55 to 75 %(w/v);
(b) preparing a second solution in which albumin is dissolved at a concentration of 15 to 25 %(w/v);
(c) mixing the first solution and the second solution to prepare a suspension;
(d) diluting the suspension with an aqueous solvent in an amount of 3 to 12 times the volume of the suspension; and
(e) drying the diluted solution to obtain nanoparticles,
wherein the taxane is paclitaxel, docetaxel, or a combination thereof, and
wherein the nanoparticles maintain an average particle size D₅₀ of 100 to 200 nm from before the drying is conducted to after the drying is completed, and wherein D₅₀ means that 50% of the particles are smaller than the average particle size.

## Patentansprüche

1. Verfahren zur Herstellung von Nanopartikeln, die Taxan und Albumin enthalten, wobei das Verfahren folgende Schritte umfasst:
(a) Herstellen einer ersten Lösung, in der Taxan in einer Konzentration von 55 bis 75 % (Gew./Vol.) gelöst ist;
(b) Herstellen einer zweiten Lösung, in der Albumin in einer Konzentration von 15 bis 25 % (Gew./Vol.) gelöst ist;
(c) Mischen der ersten Lösung und der zweiten Lösung zur Herstellung einer Suspension;
(d) Verdünnen der Suspension mit einem wässrigen Lösungsmittel in einer Menge, die das 3- bis 12-fache des Volumens der Suspension beträgt; und
(e) Trocknen der verdünnten Lösung, um Nanopartikel zu erhalten,
wobei das Taxan Paclitaxel, Docetaxel oder eine Kombination davon ist.

2. Verfahren nach Anspruch 1, wobei die Nanopartikel in der verdünnten Lösung von vor der Durchführung der Trocknung bis nach Abschluss der Trocknung eine durchschnittliche Größe D₅₀ von 100 bis 200 nm beibehalten, und wobei D₅₀ bedeutet, dass 50 % der Partikel kleiner als die durchschnittliche Partikelgröße sind.

3. Verfahren nach Anspruch 1, wobei die Nanopartikel in der verdünnten Lösung von vor der Durchführung der Trocknung bis nach Abschluss der Trocknung eine durchschnittliche Größe D₅₀ von 110 bis 190 nm beibehalten, und wobei D₅₀ bedeutet, dass 50 % der Partikel kleiner als die durchschnittliche Partikelgröße sind.

4. Verfahren nach Anspruch 1, wobei das Albumin Human-Serumalbumin (HSA), Rinder-Serumalbumin (BSA), Ovalbumin (OVA) oder eine Kombination davon ist.

5. Verfahren nach Anspruch 1, wobei die erste Lösung und die zweite Lösung in Schritt (c) in einem Volumenverhältnis von 1:20 bis 1:45 gemischt werden.

6. Verfahren nach Anspruch 1, wobei die Suspension in Schritt (c) durch Hochdruckhomogenisierung hergestellt wird.

7. Verfahren nach Anspruch 6, wobei die Hochdruckhomogenisierung bei einer Temperatur von 10 bis 40 °C durchgeführt wird.

8. Verfahren nach Anspruch 6, wobei die Hochdruckhomogenisierung bei einem Druck von 68,95 MPa bis 206,84 MPa (10.000 bis 30.000 psi) durchgeführt wird.

9. Taxan und Albumin enthaltendes Nanopartikel, hergestellt nach einem der Verfahren nach den Ansprüchen 1 bis 8.

10. Verfahren zur Stabilisierung der Partikelgrößenverteilung von Taxan und Albumin enthaltenden Nanopartikeln, wobei das Verfahren folgende Schritte umfasst:
(a) Herstellen einer ersten Lösung, in der Taxan in einer Konzentration von 55 bis 75 % (Gew./Vol.) gelöst ist;
(b) Herstellen einer zweiten Lösung, in der Albumin in einer Konzentration von 15 bis 25 % (Gew./Vol.) gelöst ist;
(c) Mischen der ersten Lösung und der zweiten Lösung zur Herstellung einer Suspension;
(d) Verdünnen der Suspension mit einem wässrigen Lösungsmittel in einer Menge, die das 3- bis 12-fache des Volumens der Suspension beträgt; und
(e) Trocknen der verdünnten Lösung, um Nanopartikel zu erhalten,
wobei das Taxan Paclitaxel, Docetaxel oder eine Kombination davon ist, und
wobei die Nanopartikel eine durchschnittliche Partikelgröße D₅₀ von 100 bis 200 nm von vor der Durchführung der Trocknung bis nach Abschluss der Trocknung beibehalten, und wobei D₅₀ bedeutet, dass 50 % der Partikel kleiner als die durchschnittliche Partikelgröße sind.

## Revendications

1. Procédé de fabrication de nanoparticules contenant du taxane et de l'albumine, le procédé comprenant les étapes suivantes :
(a) préparation d'une première solution dans laquelle du taxane est dissous à une concentration de 55 à 75 % (m/v),
(b) préparation d'une deuxième solution dans laquelle de l'albumine est dissoute à une concentration de 15 à 25 % (m/v),
(c) mélange de la première solution et de la deuxième solution pour préparer une suspension,
(d) dilution de la suspension avec un solvant aqueux dans une quantité correspondant à 3 à 12 fois le volume de la suspension, et
(e) séchage de la solution diluée pour obtenir des nanoparticules ;
le taxane étant le paclitaxel, le docétaxel ou une combinaison de ceux-ci.

2. Procédé selon la revendication 1, dans lequel les nanoparticules de la solution diluée conservent une taille moyenne D₅₀ de 100 à 200 nm avant l'exécution du séchage et après l'exécution du séchage, D₅₀ signifiant que 50 % des particules sont plus petites que la taille moyenne des particules.

3. Procédé selon la revendication 1, dans lequel les nanoparticules de la solution diluée maintiennent une taille moyenne D₅₀ de 110 à 190 nm avant l'exécution du séchage et après l'exécution du séchage, D₅₀ signifiant que 50 % des particules sont plus petites que la taille moyenne des particules.

4. Procédé selon la revendication 1, dans lequel l'albumine est de l'albumine sérique humaine (ASH), de l'albumine sérique bovine (ASB), de l'ovalbumine (OVA) ou une combinaison de celles-ci.

5. Procédé selon la revendication 1, dans lequel la première solution et la deuxième solution de l'étape (c) sont mélangées selon un rapport volumique de 1:20 à 1:45.

6. Procédé selon la revendication 1, dans lequel la suspension de l'étape (c) est préparée par homogénéisation à haute pression.

7. Procédé selon la revendication 6, dans lequel l'homogénéisation à haute pression s'effectue dans des conditions de température de 10 à 40 °C.

8. Procédé selon la revendication 6, dans lequel l'homogénéisation à haute pression est exécutée dans des conditions de pression de 68,95 MPa à 206,84 MPa (10000 à 30000 psi).

9. Nanoparticule contenant du taxane et de l'albumine, fabriquée suivant le procédé selon l'une quelconque des revendications 1 à 8.

10. Procédé de stabilisation de la distribution granulométrique de nanoparticules contenant du taxane et de l'albumine, le procédé comprenant les étapes suivantes :
(a) préparation d'une première solution dans laquelle du taxane est dissous à une concentration de 55 à 75 % (m/v),
(b) préparation d'une deuxième solution dans laquelle de l'albumine est dissoute à une concentration de 15 à 25 % (m/v),
(c) mélange de la première solution et de la deuxième solution pour préparer une suspension,
(d) dilution de la suspension avec un solvant aqueux dans une quantité correspondant à 3 à 12 fois le volume de la suspension, et
(e) séchage de la solution diluée pour obtenir des nanoparticules ;
le taxane étant le paclitaxel, le docétaxel ou une combinaison de ceux-ci, et
les nanoparticules conservant une taille moyenne de particule D₅₀ de 100 à 200 nm avant l'exécution du séchage et après l'exécution du séchage, D₅₀ signifiant que 50 % des particules sont plus petites que la taille moyenne des particules.
